# EUROPEAN PATENT SPECIFICATION

(11) **EP 2 303 911 B1**
(45) Date of publication and mention of the grant of the patent: **15.08.2012**
(21) Application number: 08874714.2
(22) Date of filing: 15.10.2008
(51) Int. Cl.: C07K 7/00, C07K 14/51

(54) **A SYNTHETIC PEPTIDE CONSISTING OF BONE FORMING PEPTIDE 2(BFP 2) FOR STIMULATING OSTEOBLAST DIFFERENTIATION, AND PHARMACEUTICAL COMPOSITION COMPRISING THE SYNTHETIC PEPTIDE**
EIN BONE FORMING PEPTIDE 2 (BFP 2) DARSTELLENDES SYNTHETISCHES PEPTID ZUR STIMULIERUNG DER OSTEOBLASTENDIFFERENZIERUNG, UND EINE DAS SYNTHETISCHE PEPTID ENTHALTENDE PHARMAZEUTISCHE ZUSAMMENSETZUNG
PEPTIDE SYNTHÉTIQUE CONSISTANT EN UN PEPTIDE DE FORMATION OSSEUSE 2 (BFP2) POUR STIMULER UNE DIFFÉRENCIATION OSTÉOBLASTIQUE, ET COMPOSITION PHARMACEUTIQUE COMPRENANT LE PEPTIDE SYNTHÉTIQUE

(30) Priority: 20.06.2008 KR 20080058551
(43) Date of publication of application: 06.04.2011
(73) Proprietor: Industry Foundation Of Chonnam National University, Gwangju 500-757 (KR)
(72) Inventor: YOON, Taek Rim, Gwangju 501-833 (KR); KIM, Hyung Keun, Hwasun-gun Jeollanam-do 519-752 (KR); KIM, Ji Hyun, Gwangju 503-051 (KR)
(74) Representative: Beck & Rössig European Patent Attorneys
(86) International application number: PCT/KR2008/006059
(87) International publication number: WO 2009/154330

(56) References cited:
- WO-A1-90/11366
- WO-A2-2006/073711
- US-A- 5 693 615
- DATABASE EPO Proteins [Online] 29 April 2010 (2010-04-29), "Sequence 12866 from Patent WO2010037395.", XP002639956, retrieved from EBI accession no. EPOP:HC534090 Database accession no. HC534090
- LI T. ET AL.: 'Bone morphogenetic protein 7: a novel treatment for chronic renal and bone disease' CURR OPIN NEPHROL HYPERTENS. vol. 13, no. 4, July 2004, pages 417 - 422, XP002565669

<110> INDUSTRY FOUNDATION OF CHONNAM NATIONAL UNIVERSITY et al
<120> An synthetic peptide containing bone forming peptide 2(BFP 2) for stimulating osteoblast differentiation, and pharmaceutical composition comprising the synthetic peptide
<130> FO8PCT1023
<150> KR10-2008-58551
   <151> 2008-06-20
<160> 1
<170> KopatentIn 1.71
<210> 1
   <211> 15
   <212> PRT
   <213> Artificial Sequence
<220>
   <223> Bone forming peptide 2(BFP2) is derived from BMP-7.
<400> 1
   Val Glu His Asp Lys Glu Phe Phe His Pro Arg Tyr His His Arg
    1               5                  10                  15

## Description

### Technical Field

The present invention relates to a synthetic peptide derived from BMP-7 (bone morphogenetic protein-7) and consisting of 15 amino acids, and a pharmaceutical composition and a medium composition comprising the same.

### Background Art

Osteoporosis is a disease of bone that leads to an increased risk of fracture because the bone mineral density (BMD) is reduced, bone microarchitecture is disrupted and the medullary cavity is enlarged.

The bone mass, accounting for the state of health of the bone, is dependent on various factors, such as heredity, nutrition, hormonal changes, exercise, life style, etc. Particularly, aging, shortage of exercise, low body weight, smoking, a low-calcium diet, menopause, or ovary resection are known to be causes of osteoporosis.

Because of the lower bone resorption level thereof, black people are generally higher in bone mass than are white people although there are differences between individuals. On the whole, the bone mass peaks at fourteen to eighteen years of age, and decreases by 1% per year in old age. Particularly, women over 30 years old continuously decrease in bone mass. Hormonal change at menopause causes the bone mass to rapidly decrease. In detail, women in menopause experience a rapid decrease of estrogen, which allows the production of an increased level of B-lymphocytes and the accumulation of pre-B cells in the bone marrow, causing an increase in IL-6 level. IL-6 acts to activate osteoclasts, thus decreasing the bone marrow.

Osteoporosis, as described above, inevitably accompanies senescence, especially post-menopausal women. Entering senescent societies, advanced countries have increasingly paid attention to osteoporosis and therapy therefor.

In addition to osteoporosis, osteoarthritis and bone defective disease are representative bone diseases. Osteoarthritis is known as degenerative arthritis entailing local degradation of joints including articular cartilage and the subchondral bone next to it. Osteoarthritis has two typical causes: when an excessive load is imposed to the joint although articular cartilage or the subchondral bone is normal and when articular cartilage or the subchondral bone is weak, and even though a proper load is imposed to the joint.

Bone defects may be found in many sites of the body. Its main causes include acute trauma with the accompaniment of ossein loss, a tissue resection operation accompanied by bone loss, chronic inflammation with the accompaniment of bone resection, and chronic nonunion with the accompaniment of the segmental bone defects.

As concerns the medicine for bone diseases, the world market thereforamounts to about one hundred thirty billion dollars and is expected to gradually expand. Thus, leading research institutes and pharmaceutical companies have invested a lot of money in developing therapeutics for bone diseases.

For example, currently used therapeutics for osteoporosis include estrogen, androgenic anabolic steroids, calcium agents, phosphates, fluorine agents, Ipriflavone, and vitamin D3. Additional examples include aminobisphosphonate, developed by Merck in 1995, and raloxifene, acting as a selective estrogen receptor modulator (SERM), developed by Lilly Co. in 1997.

Acting as a negative regulator of bone resorption, estrogen is now most widely used as a therapeutic for osteoporosis, but the habitual use thereof may increase the incidence of endometrial cancer and breast cancer and cause thrombosis, gallstones, hypertension, edema, and mastodynia. A group of studies shows that menopausal women co-administered with estrogen and progesterone arehighly apt to suffer from breast cancer, stroke, and pulmonary embolism.

Turning to other therapeutics for osteoporosis, there are bisphosophonate agents (alendronate, etidronate), vitamin D agents, calcitonin agents, and calcium agents. However, bisphosphonate agents cause esophagitis in addition to being low in adsorptivity and difficult to take.

Vitamin D agents are expensive and have pharmaceutical effects which are still unknown. Calcitonin agents are expensive and difficult to take. Calcium agents, although with low side effects, are preventive of rather than therapeutic for osteoporosis.

LI T. ET AL.: 'Bone morphogenetic protein 7: a novel treatment for chronic renal and bone disease' CURR OPIN NEPHROL HYPERTENS. vol. 13, no. 4, July 2004, pages 417 - 422 disclose BMP-7 and its use in the treatement of bone diseases.

Further, osteoporosis cannot be cured in the short term, but requires long-term administration of drugs. Accordingly, there is a need for a novel material which shows none of the above-mentioned side effects and is therapeutically effective enough to replace conventional drugs.

Furthermore, the prophylaxis and treatment of osteoporosis still remains to be improved. Particularly, the secondary osteoporosis resulting from drug abuse becomes aggravated.

Such situations are true of osteoarthritis and bone defects.

### Technical Problem

Leading to the present invention, intensive and thorough research into therapeutics for osteoporosis, conducted by the present inventors, aiming to overcomethe problems encountered in the prior art, resulted in the finding that a peptide derived from BMP-7 is promotive of osteoblast differentiation.

It is therefore an object of the present invention to provide a synthetic peptide, which promotes osteoblast differentiation or osteogenesis and can be synthesized at a low cost.

It is another object of the present invention to provide a pharmaceutical composition comprising the osteogenic synthetic peptide as an active ingredient, which is effective for the treatment of osteoporosis, osteoarthritis and/or bone defects, without side effects and producible at low cost.

It is a further object of the present invention to provide a medium composition for osteoblast differentiation, comprising the osteogenic synthetic peptide, with which the rate of osteoblast differentiation or bone formation can be controlled according to the intention of the user.

It should be noted that the objects of the present invention are not limited to the above-mentioned objects, and other non-mentioned objects can be clearly understood to those skilled in the art from the following description.

### Technical Solution

In order to accomplish the above objects, the present invention provides an osteogenic synthetic peptide consisting of the amino acid sequence of SEQ ID NO. 1 [bone forming peptide 2(BFP 2)]:
SEQ ID NO. 1:
Val-Glu-His-Asp-Lys-Glu-Phe-Phe-His-Pro-Arg-Tyr-His-His-Arg

Advatageous embodiments are indicated in further claims.

In a preferred embodiment, an effective dose of the synthetic peptide for promoting osteoblast differentiation or bone formation is from 0.1 to 2 µg/ml.

In addition, the present invention provides a pharmaceutical composition comprising the osteogenic synthetic peptide as an active ingredient for treatment of osteoporosis, osteoarthritis, or bone defects.

Also, the present invention provides an osteogenic differentiation medium, comprising said osteogenic synthetic peptide.

### [Advantageous Effects]

The advantageous effects of the present invention are as follows.

The osteogenic synthetic peptide BFP 2 of the present invention can be usable in various field, because the synthetic peptide BFP 2 is effective for promoting osteoblast differentiation or osteogenesis, and can be synthesized at a low cost.

The pharmaceutical composition comprising the osteogenic synthetic peptide BFP 2 as an active ingredient of the present invention is effective for the treatment of osteoporosis, osteoarthritis and/or bone defects, without side effects and producible at low cost

The medium composition for osteoblast differentiation comprising the osteogenic synthetic peptide BFP 2 of the present invention can be controlled with the rate of osteoblast differentiation or bone formation according to the intention of the user.

### [Description of Drawings)

The above and other objects, features and advantages of the present invention will be more clearly understood from the following detailed description taken in conjunction with the accompanying drawings, in which:
FIG. 1 shows amino acid sequences of BFP 2, the osteogenic synthetic peptide of the present invention;
FIG 2 is photographs taken of monitor screens on which structures of BFP 2 is displayed;
FIG. 3 is a histogram showing the cytotoxicity of BFP 2;
FIG. 4 is photographs showing the mineralization visualized by alizarin red staining during osteoblast differentiation in the presence of BFP 2;
FIG. 5 is of fluorescence microphotographs showing the expression of the osteoblast-specific osteocalcin gene and the osteoblast-specific alkaline phosphatase (ALP) gene in the presence of BFP 2;
FIG. 6 shows FACS cytometry analysis of the expression of cell surface markers accounting for osteoblast differentiation in the presence of BFP 2 (grey arrows ODM alone; black arrows ODM + BFP 2);
FIG. 7 is fluorescence microphotographs showing the expression of the cell surface marker CD44 in the presence of BFP 2;
FIG. 8 is histograms showing the effect of BFP 2 on the expression of the osteoblast-specific alkaline phosphatase (ALP) and mineralization as determined by a commercially available kit;
FIG. 9 is of fluorescence microphotographs showing the expression of the osteoblast-specific Runx-2 gene in the presence of BFP 2;
FIG. 10 is of histograms showing the migration of cells to BFP 2, via a chemotaxis assay during differentiation from mesenchymal stem cells to osteoblasts.

### [Best Mode]

The terminology used in the present invention is taken from the most widely used general terms, but in particular cases, it may come from the thesaurus of the present inventors.It should be noted that the meanings of the terms used in the specification and the following claims must be understood in consideration of the detailed description of the present invention or in association with the total context of the specification, and are not to be understood without reference thereto.

Below, a detailed description will be given of the structural constitution of the present invention, with reference to the following drawings.

As concerns bone morphogenetic activity, it was found that bone morphogenetic proteins (BMPs) which belong to the TGF- β (transforming growth factor-β) superfamily of proteins (Science 150, 893-897, 1965; Science 242; 1528-1534, 1988) are responsible for it. Well known are fourteen BMPs named BMP-1 to BMP-14. Of them, BMP-2 to BMP-14 are known to have bone morphogenetic activity. Although considered effective for the treatment of various bone dysfunctions and bone diseases, BMP-2 to BMP-14are present in trace amounts in nature. Thus, the production of a large amount of BMP-2 to BMP-14 has to make recourse to a recombinant technique. In general, the production of recombinant proteins is expensive as compared to low-molecular weight compounds. In addition, the proteinous characteristics thereof bring about many limitations to the use of BMPs as drugs in terms of properties and administration. In consideration of these situations, a low-molecular weight organic compound having activity identical to that of BMP, if present, may be a promising drug.

As a result of intensive study into the amino acid sequences of BMP-7, the present inventors found that the following amino acid sequence derived from BMP-7, consisting of 15 amino acid residues, induce the expression of human BMP-7 in addition to having activity identical to that of human BMP-7.

It was named bone forming peptide 2 (BFP 2).
SEQ ID NO. 1:
Val-Glu-His-Asp-Lys -Glu-Phe-Phe-His-Pro-Arg-Tyr-His-His-Arg

Although derived from BMP-7, BFP 2 consisting of 15 amino acid residues, can be synthesized using a known peptide synthesis method.

As will be clearly elucidated in the following experimental examples, osteoblasts (more accurately, mouse mensenchymal stem cells) are more intensively induced into differentiation when in the presence of BFP 2.

Hence, the synthetic peptide for promoting osteogenesis, having a BMP-7-derived amino acid sequence of 15 amino acid residues, that is, BFP 2 enhances the differentiation of osteoblasts into mature bone cells, thus showing osteogenesis promoting activity.

From the fact that the synthetic peptide promoting osteogenesis in accordance with the present invention has excellent activity related to inducing the differentiation of osteoblasts, it can be inferred that when bone loss is caused by a bone disease such as osteoporosis, osteoarthritis or a bone defect, a pharmaceutical composition comprising as an active ingredient the synthetic peptide of the present invention or a non-toxic salt thereof may be applied to the site of bone loss so as to treat osteoporosis, osteoarthritis and/or the bone defect.

With reference to the accompanying drawings, an explanation is given of the activity of the osteogenesis-promoting synthetic peptides derived from BMP-7 in accordance with the present invention, BFP 2, to induce osteoblast differentiation.

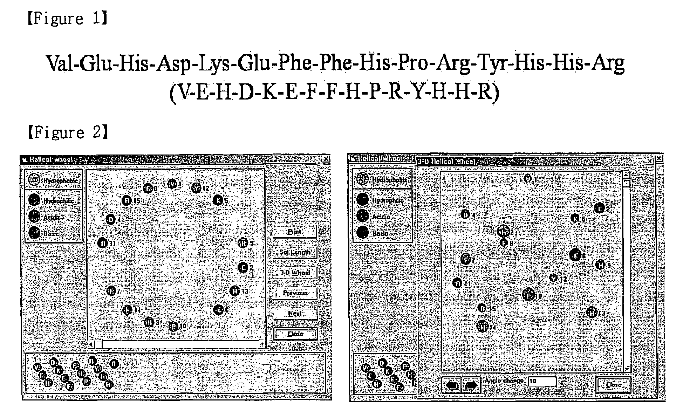

FIGS. 1 to FIG. 3 show amino acid sequence, structure and cytotoxicity of BFP 2.

BFP 2 shown in FIG. 1 is identical to partial amino acid sequences of BMP-7 and can be synthesized to have 15-mer peptide sequence of SEQ ID NO. 1.

Referring to FIG. 2, structure of the synthetic peptide BFP 2 is shown on the display terminals as reproduced by a commercially available program. As shown, the peptide sequences of FIG. 1 is of alpha-helix structure.

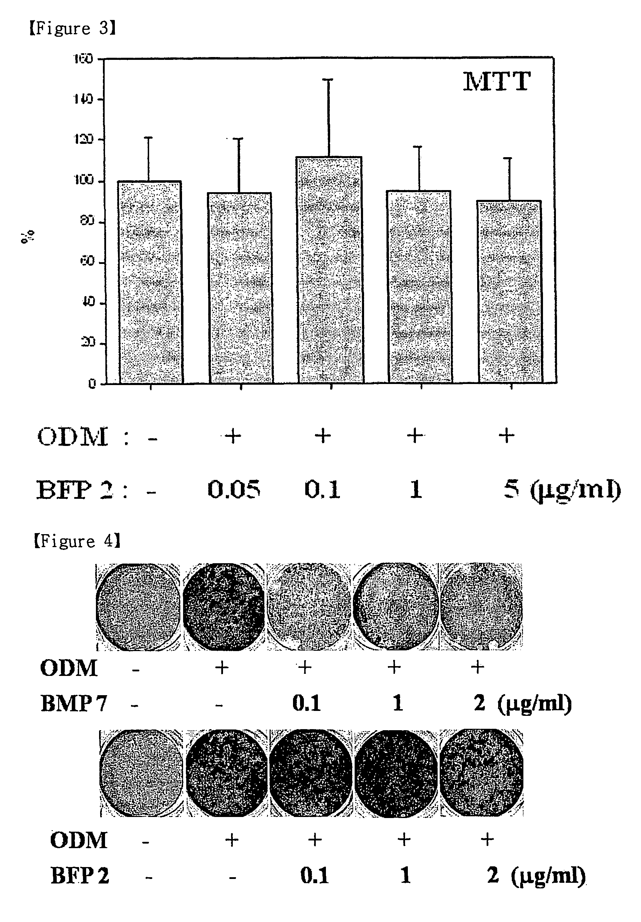

An MTT assay for cytotoxicity shows that BFP 2 is not toxic to cells within a range of used concentrations, as shown in FIG. 3.

A better understanding of the present invention may be obtained through the following examples which are set forth to illustrate, but are not to be construed as limiting the present invention.

### [Mode for Invention]

### EXPERIMENTAL EXAMPLE 1

The following experiments were conducted to assay BFP 2 for activity related to the differentiation of osteoblasts.

### 1. Preparation of Osteoblasts and Osteogenic Differentiation Medium

Mesenchymal stem cells (cloned from Balb/c mouse bone marrow stromal cells) were seeded at a density of 1 x 10⁴ cells/well in DMEM supplemented with 10% FBS on plates, followed by incubation for 3 days at 37° C in a 5% CO₂ atmosphere to prepare osteoblasts.

An osteogenic differentiation medium (hereinafter referred to as "ODM") was prepared by supplementing DMEM with 50 µg/ml ascorbic acid, 10⁻⁸ M dexamethasone and 10 mM beta-glycerocellophosphate.

### 2. Assay for Mineralization

When the mesenchymal stem cells differentiate into osteoblasts, calcium is accumulated. Thus, the accumulation of calcium ions signals osteoblast differentiation and can be quantitatively detected through alizarin red staining because Alizarin red staining appears as gross and microscopic observation of osteoblasts differentiated from mesenchymal stem cells.

Therefore, cells treated with BFP 2 in osteogenic differentiation media can be assayed for osteoblast differentiation by observing alizarin red staining.

In more detail, mesenchymal stem cells were cultured for three days in an osteogenic differentiation medium and then for an additional two days in the presence of BFP 2 at a concentration of 0.1 µg/ml, 1 µg/ml and 2 µ g/ml.

Afterwards, the cells were fixed for one hour with ice-cold 70% ethanol and stained for 10 min with an alizarin red-s solution to observe calcium accumulation, that is, accumulation in terms of red color. The result is shown in FIG. 4.

From the data of FIG. 4 which show alizarin red-stained cells treated with the BMP-7-derived, osteogenic syntheticpeptides BFP 2, accounting for osteoblast differentiation in terms of mineralization, the most intense colors were observed in the cells treated with BFP 2 at a concentration of 1 µg/ml. Also, more intense colors appeared in the cells treated with BFP 2 than with those treated with BMP-7, indicating that BFP 2 is more effective in inducing osteoblast differentiation than is BMP-7.

### EXPERIMENTAL EXAMPLE 2

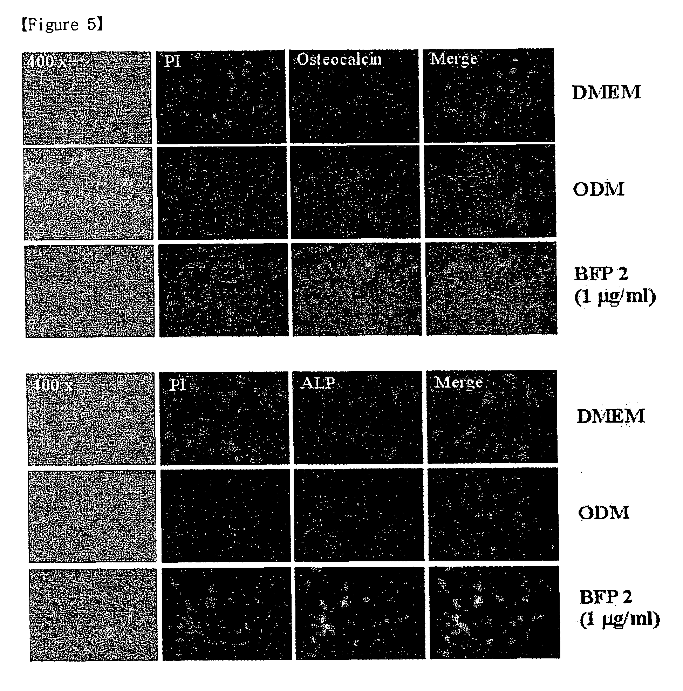

An examination was made of the effects of BPF 2 on osteoblast differentiation. For this, the expression levels of osteoblast -specific genes, osteocalcin and alkaline phosphatase were detected using fluorescent antibodies thereto and observed under a fluorescence microscope. The results are successively given in FIG. 5.

In detail, differentiation into osteoblasts was accompanied by the expression of osteoblast-specific proteins which were then reacted with antibodies thereof. The antibodies bound to the proteins were observed under a fluorescence microscope. The fluorescence microscopy showed that osteocalcin, a bone matrix material secreted from osteoblasts whose higher levels are well correlated with an increase in the growth and differentiation of an osteoblast, was expressed at a higher level in an osteogenic differentiation medium (ODM) supplemented with 1 µg/ml of BFP 2 than in an osteogenic differentiation alone or supplemented with BMP-7 as observed by the more intense green color (brighter portions in the drawings).

In FIG. 5, the PI panels are of stained nuclei of living cells, indicating living cells while the target protein osteocalcin appeared green from the cells due to the fluorescent antibodies thereof. The merge panels, resulting from overlapping the PI panels with the target protein panels, show the expression of the target protein from the same cells.

Expressed in a mid-phase of osteoblast differentiation, alkaline phosphatase serves as a biomarker for bone formation. Alkaline phosphatase was found to be expressed at higher levels in the presence of 1 µg/ml of BFP 2, as well.

### EXPERIMENTAL EXAMPLE 3

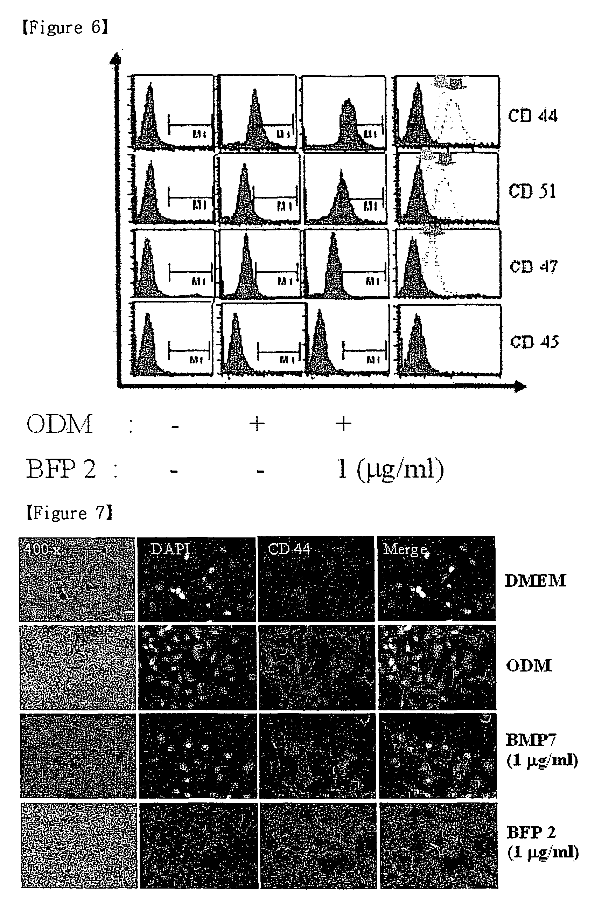

Having various surface proteins, mesenchymal stem cells can differentiate into various cell types including osteoblasts, chondrocytes, adipocytes, myocytes, tendons, ligaments, neurons, and blood vessels. The effect of BFP 2 on the differentiation of mesenchymal stem cells into osteoblasts was analyzed by FACS for the expression of osteoblast-specific surface proteins CD44, CD51, CD47 and CD45. The results are shown in FIG. 6.

With reference to FIG. 6, the expression of the surface markers accounting for osteoblast differentiation was analyzed using FACS (gray arrows ODM alone; black arrows ODM + BFP 2).BFP 2 was observed to induce the expression of CD44, a surface marker for differentiation from mensenchymal stem cells to osteoblasts. Particularly in the presence of BFP 2 at a concentration of 1 µg/ml in an osteogenic differentiation medium, CD44 was expressed at a higher level. On the other hand, CD47 and CD51, both specific for osteoblasts, were observed to be expressed at higher levels in an ODM supplemented with BFP 2 than in an ODM alone.

In addition, CD51, specific for osteoblasts, was observed to be expressed at higher levels in an ODM supplemented with BFP 2 than in an ODM alone.

### EXPERIMENTAL EXAMPLE 4

The effect of BFP 2 on the expression of the surface marker CD44 was observed under a fluorescence microscope and the results are given in FIG. 7.

During differentiation, the cells were stained with DAPI, a fluorescent stain that easily binds to the DNA of living cells, and with a red anti-CD44 antibody.

The nuclei appeared blue while CD44 was stained red under fluorescence microscopy. When these stained images were merged, it was observed that CD44 was expressed on the DAPI-stained cells. Higher levels of CD44 were detected when an osteogenic differentiation medium was used in BFP 2 than when used alone. At the same concentrations, BMP-7 produced less intense staining in the cells than did BFP 2 under a fluorescence microscope.

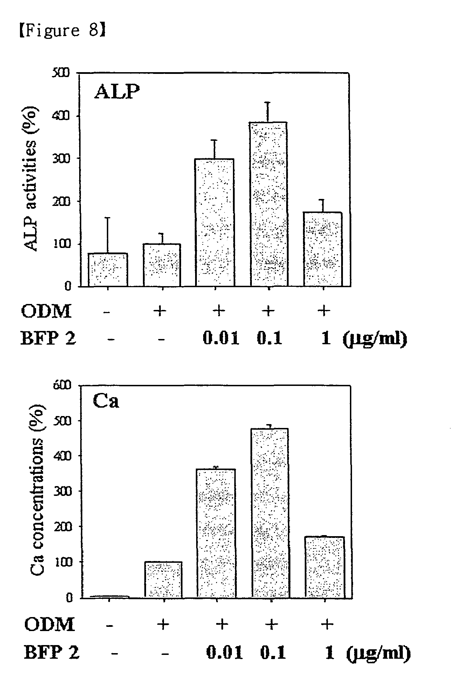

When osteoblast differentiation was permitted in the presence of BFP 2 , the concentrations of osteoblast-specific alkaline phosphatase (ALP) and calcium were measured using a commercially available diagnostic kit. The results are depicted in FIG. 8.

Significant increases in the levels of both ALP and calcium were observed in the presence of BFP 2 at a concentration of 1 µg/ml, as seen in FIG. 8.

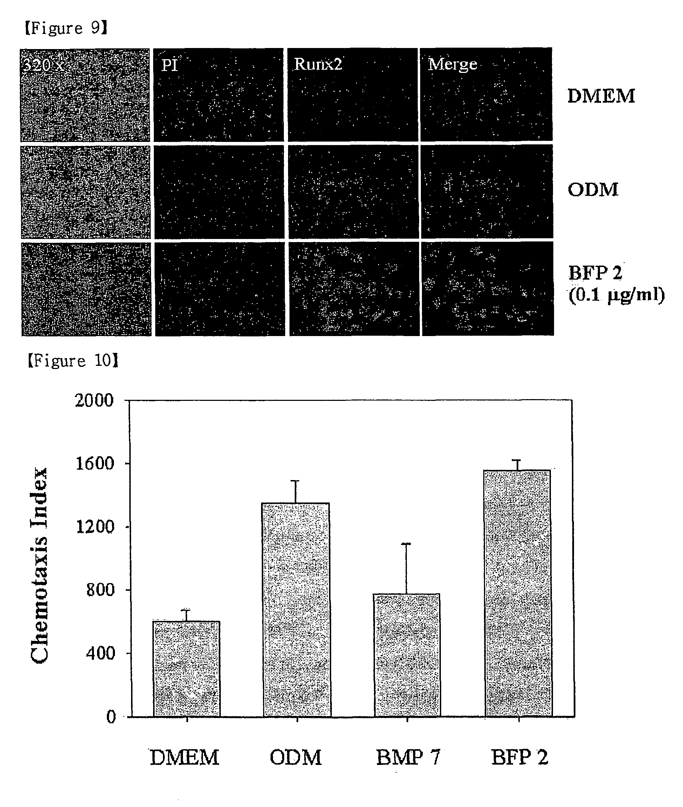

FIG. 9 is of fluorescence microphotographs showing the expression of the osteoblast-specific Runx-2 gene in the presence of BFP 2.

Runx-2, as a transcription factor of an osteoblast-specific protein, plays an important role in osteoblast differentiation. In FIG. 9, a higher expression level of Runx-2 was also detected in the presence of 0.1 µg/ml of BFP 2 than in the presence of 0.1 µg/ml of BMP-7.

While differentiating into osteoblasts, BFP 2 was added to mesenchymal stem cells to examine chemotaxis and cell migration. The results are shown in FIG. 10.

For this, mesenchymal stem cells were incubated in osteogenic differentiation media supplemented with BMP-7, BFP 2. As seen in FIG. 10, more cells were observed to migrate in the presence of BFP 2 than in the presence of BMP-7, indicating that BFP 2 according to the present invention can induce osteoblasts to rapidly migrate to lesions of bone diseases such as bone defects.

From the data, it is apparently understood that the osteogenic synthetic peptide of the present invention, BFP 2, plays a more important role in osteoblast differentiation than does BMP-7, known as an osteogenesis promoter, and can induce osteogenesis to the same or a higher extent and over a broader range than can BMP-7.

Therefore, the present invention provides a pharmaceutical composition comprising the osteogenic synthetic peptide of the present invention or a non-toxic salt thereof in combination with a pharmaceutically or veterinarilly acceptable liquid or solid carrier. As long as it is known in the art, any pharmaceutically or veterinarilly acceptable liquid or solid carrier can be used in the present invention without limitation. Thus, a detailed description is not given of the carrier.

As a result, the pharmaceutical composition of the present invention may be used as a therapeutic for osteoporosis and/or osteoarthritis.

Likewise, when applied to bone defective sites, the pharmaceutical composition comprising the osteogenic synthetic peptide, BFP 2, or a non-toxic salt thereof can effectively conduct bone restoration through bone formation.

Although specified as examples, it is apparent to those in the art that when added to osteogenic differentiation media, the osteogenic synthetic peptide according to the present invention, BFP 2, allows the user to easily control the experiments with osteoblasts.

Being able to promote osteoblast differentiation and thus bone formation, as described hitherto, the osteogenic synthetic peptide/ of the present invention finds useful applications in various fields related with osteoblast differentiation or bone formation.

In addition, the pharmaceutical composition comprising the osteogenic synthetic peptide in accordance with the present invention is useful in the treatment of osteoporosis, osteoarthritis and/or bone defects with few side effects compared to conventional pharmaceutical compositions and is effective in promoting bone formation through the promotion of osteoblast differentiation in addition to being economically favorable thanks to the low production cost thereof.

Further, the medium composition containing the osteogenic synthetic peptide of the present invention allows the user to control the rate of osteoblast differentiation thereby enabling the set up of a variety of experimental conditions.

Although the preferred embodiments of the present invention have been disclosed for illustrative purposes, those skilled in the art will appreciate that various modifications, additions and substitutions are possible, without departing from the scope of the invention as disclosed in the accompanying claims.
## Claims

1. An osteogenic synthetic peptide consisting of the amino acid sequence of SEQ ID NO. 1 [bone forming peptide 2(BFP 2)]:
SEQ ID NO. 1:
Val-Glu-His-Asp-Lys-Glu-Phe-Phe-His-Pro-Arg-Tyr-His-His-Arg.

2. A pharmaceutical composition comprising the osteogenic synthetic peptide of claim 1 or a non-toxic salt thereof alone or in combination with a pharmaceutically or veterinarilly acceptable liquid or solid vehicle.

3. The pharmaceutical composition of claim 2, wherein the osteogenic synthetic peptide or the non-toxic salt thereof is used as an active ingredient for treatment of osteoporosis,
osteoarthritis, or bone defects.

4. The pharmaceutical composition of claim 2 or 3, wherein an effective dose of the synthetic peptide for promoting osteoblast differentiation or bone formation is from 0.1 to 2 µg/ml.

5. An osteogenic differentiation medium comprising the osteogenic synthetic peptide of claim 1.

## Patentansprüche

1. Osteogenes synthetisches Peptid, bestehend aus der Aminosäuresequenz SEQ ID Nr. 1 [knochenformendes Peptid 2 (BFP 2)]:
SEQ ID Nr. 1:
Val-Glu-His-Asp-Lys-Glu-Phe-Phe-His-Pro-Arg-Tyr-His-His-Arg.

2. Pharmazeutische Zusammensetzung, die das osteogene synthetische Peptid nach Anspruch 1 oder ein nicht-toxisches Salz daraus allein bzw. in Kombination mit einer pharmazeutisch oder veterinärmedizinisch annehmbaren flüssigen oder feststofflichen Trägersubstanz enthält.

3. Pharmazeutische Zusammensetzung nach Anspruch 2, wobei das synthetische Peptid oder das nicht-toxische Salz daraus als Wirkstoff zur Behandlung von Osteoporose, Osteoarthritis oder Knochendefekten verwendet wird.

4. Pharmazeutische Zusammensetzung nach Anspruch 2 oder 3, wobei eine wirksame Menge des synthetischen Peptids zur Osteoblasten-Differenzierung oder Knochenbildung zwischen 0,1 und 2 µg/ml liegt.

5. Medium zur osteogenen Differenzierung, welches das osteogene synthetische Peptid nach Anspruch 1 umfasst.

## Revendications

1. Peptide synthétique ostéogénique consistant en la séquence d'amino-acides de SEQ ID NO.1 [(peptide de formation osseuse 2 (BFP2)] :
SEQ ID NO. 1 :
Val-Glu-His-Asp-Lys-Glu-Phe-Phe-His-Pro-Arg-Tyr-His-His-Arg.

2. Composition pharmaceutique comprenant le peptide synthétique ostéogénique de la revendication 1 ou un sel non toxique de celui-ci seul ou en combinaison avec un véhicule liquide ou solide acceptable sur le plan pharmaceutique ou vétérinaire.

3. Composition pharmaceutique selon la revendication 2 dans laquelle le peptide synthétique ou le sel non toxique de celui-ci est utilisé comme un ingrédient actif pour le traitement de l'ostéoporose, de l'ostéoarthrite ou des déficits osseux.

4. Composition pharmaceutique selon la revendication 2 ou 3 dans laquelle une quantité efficace de peptide synthétique pour favoriser la différenciation des ostéoblastes ou la formation osseuse est de 0,1 à 2 µg/ml.

5. Milieu de différenciation ostéogénique comprenant le peptide synthétique ostéogénique de la revendication 1.
